# EUROPEAN PATENT APPLICATION

(11) **EP 4 184 516 A1**
(43) Date of publication of application: **24.05.2023**
(21) Application number: 20948569.7
(22) Date of filing: 07.08.2020
(51) Int. Cl.: G16H 10/40

(54) **INFORMATION PROCESSING DEVICE, INFORMATION PROCESSING METHOD, AND COMPUTER PROGRAM**

(71) Applicant: Nippon Telegraph And Telephone Corporation, Chiyoda-ku Tokyo 100-8116 (JP)
(72) Inventor: MOGAKI, Takefumi, Musashino-shi, Tokyo 180-8585 (JP); NAGATA, Kengo, Musashino-shi, Tokyo 180-8585 (JP); ITO, Hitoshi, Musashino-shi, Tokyo 180-8585 (JP)
(74) Representative: Brevalex
(86) International application number: PCT/JP2020/030332
(87) International publication number: WO 2022/029994

(57) **Abstract**

A user information management device of an embodiment includes an identifier issuance unit that issues a sample identifier for identifying a sample and a user registration identifier for identifying a user who has provided the sample, a storage unit that stores a sample identifier and a user registration identifier issued by the identifier issuance unit in association with each other, and a user information management unit that associates user information related to a user who has provided a sample identified by the sample identifier with the sample identifier in a case where the user information is provided together with the sample identifier, and associates a user identifier that is identification information of the user and is different from the user registration identifier with user information by associating the user identifier with the user registration identifier in a case where the user identifier is provided together with the user registration identifier.

## Description

### Technical Field

The present invention relates to technology for providing information related to health.

### Background Art

Conventionally, regular medical examinations such as so-called medical checkups and complete medical examinations have been conducted for company employees on the basis of related laws and regulations. While these regular medical examinations are provided to employees by companies as part of benefit programs, in recent years, service for providing information related to health to individual users (hereinafter referred to as "health service") have increased. As an example of such health service, there is service for providing users with information based on gene information such as disease or illness that tend to develop (for example, see Non Patent Literature 1).

### Citation List

### Non Patent Literature

Non Patent Literature 1: "Flow of Inspection" https://mycode.jp/howitworks.html, MYCODE.

### Summary of Invention

### Technical Problem

However, since such health service is generally provided by a business provider different from a business provider such as a medical institution at which medical examinations are performed, the health service is not necessarily highly convenient for users. For example, a user has to collect a sample for using the health service separately from collecting a sample for a medical examination (for example, blood or urine), which is troublesome. Furthermore, for example, in a case where a method and a form of providing information are different between business providers, there is a possibility that a load of acquisition and management of information increases and motivation to use the health service decreases.

In view of the above circumstances, an object of the present invention is to provide technique capable of improving convenience of health service.

### Solution to Problem

An aspect of the present invention is an information management apparatus including an identifier issuance unit that issues a sample identifier for identifying a sample and a user registration identifier for identifying a user who has provided the sample, a storage unit that stores a sample identifier and a user registration identifier issued by the identifier issuance unit in association with each other, and a user information management unit that associates user information related to a user who has provided a sample identified by the sample identifier with the sample identifier in a case where the user information is provided together with the sample identifier, and associates a user identifier that is identification information of the user and is different from the user registration identifier with user information by associating the user identifier with the user registration identifier in a case where the user identifier is provided together with the user registration identifier.

An aspect of the present invention is an information management method including an identifier issuance step for issuing a sample identifier for identifying a sample and a user registration identifier for identifying a user who has provided the sample, a storage step for storing a sample identifier and a user registration identifier issued in the identifier issuance step in association with each other, and an information management step for associating user information related to a user who has provided a sample identified by the sample identifier with the sample identifier in a case where the user information is provided together with the sample identifier, and associating a user identifier that is identification information of the user and is different from the user registration identifier with user information by associating the user identifier with the user registration identifier in a case where the user identifier is provided together with the user registration identifier.

An aspect of the present invention is a computer program that causes a computer to execute steps, the steps including an identifier issuance step for issuing a sample identifier for identifying a sample and a user registration identifier for identifying a user who has provided the sample, an identifier management step for managing a sample identifier and a user registration identifier issued in the identifier issuance step in association with each other, and an information management step for associating user information related to a user who has provided a sample identified by the sample identifier with the sample identifier in a case where the user information is provided together with the sample identifier, and associating a user identifier that is identification information of the user and is different from the user registration identifier with user information by associating the user identifier with the user registration identifier in a case where the user identifier is provided together with the user registration identifier.

### Advantageous Effects of Invention

According to the present invention, more convenient health service can be provided.

### Brief Description of Drawings

Fig. 1 is a diagram illustrating a specific example of a configuration of a health service providing system in a first embodiment.
Fig. 2 is a block diagram illustrating a specific example of a functional configuration of a user information management apparatus in the first embodiment.
Fig. 3 is a sequence diagram illustrating a flow of processing and information cooperation from collection of a sample to provision of user information in the health service providing system of the first embodiment.
Fig. 4 is a diagram illustrating an implementation example of processing and information cooperation from collection of a sample to provision of user information in the health service providing system of the first embodiment.
Fig. 5 is a diagram illustrating an implementation example of processing and information cooperation from collection of a sample to provision of user information in the health service providing system of the first embodiment.
Fig. 6 is a diagram illustrating a specific example of a configuration of a health service providing system in a second embodiment.
Fig. 7 is a block diagram illustrating a specific example of a functional configuration of a user information management apparatus in the second embodiment.
Fig. 8 is a diagram illustrating a flow of invalidation of a user registration identifier in the user information management apparatus of the second embodiment.
Fig. 9 is a diagram illustrating a flow of invalidation of both a sample identifier and a user registration identifier in the user information management apparatus of the second embodiment.

### Description of Embodiments

Embodiments of the present invention will be described in detail with reference to the drawings. Note that, in each of the following embodiments, service for acquiring and analyzing gene information on the basis of a sample provided from a user is assumed as an example of health service, but a user information management method according to the present embodiments can be applied to information management of any health service in which different business providers cooperate for a sample of a user. For example, the user information management method according to the present embodiments can also be applied to information management in service, for example, for analyzing the presence or absence of antibodies in a user body regarding infectious diseases represented by COVID-19.

### <First Embodiment>

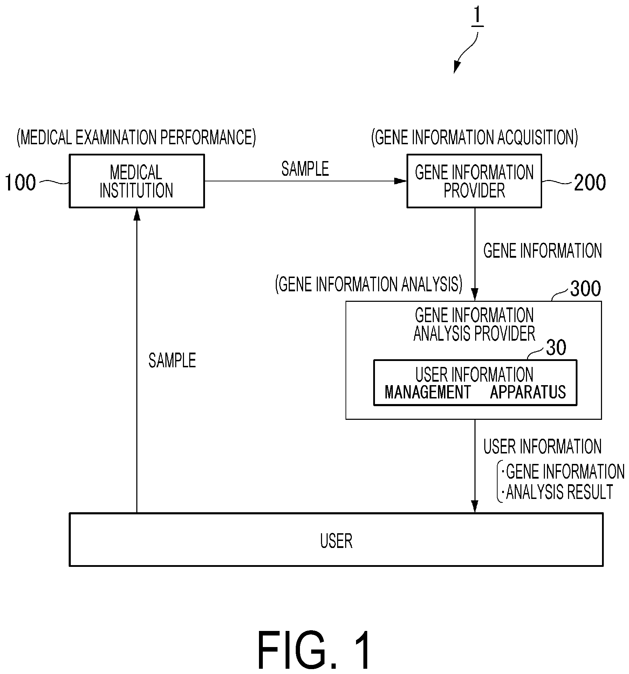

Fig. 1 is a diagram illustrating a specific example of a configuration of a health service providing system in a first embodiment. For example, a health service providing system 1 is a system in which health service providers provide health service to a user in cooperation with a medical institution 100. A gene information provider 200 and a gene information analysis provider 300 are examples of the health service providers. The medical institution 100 is a medical institution such as a hospital at which a medical examination of a user is performed. At the medical institution 100, a sample is provided from a user and a medical examination is performed on the basis of the provided sample, and a part of the provided sample is provided to the gene information provider 200.

As an example of the health service, the gene information provider 200 is a business provider that implements service for providing gene information to a user. In the present embodiment, the gene information provider 200 acquires gene information on the basis of a sample provided from the medical institution 100, and provides the acquired gene information to the gene information analysis provider 300.

As an example of the health service, the gene information analysis provider 300 is a business provider that analyzes gene information of a user and provides health advice to the user on the basis of the analysis result. For example, the gene information analysis provider 300 analyzes a physical constitution of a user on the basis of gene information, and proposes change in behavior desired for maintaining or promoting health according to the physical constitution. In the present embodiment, the gene information analysis provider 300 analyzes gene information provided from the gene information provider 200, and provides the analysis result to a user.

As described above, since the health service providing system 1 of the present embodiment provides the health service in cooperation with a plurality of business providers (that is, the medical institution 100, the gene information provider 200, and the gene information analysis provider 300), sufficient security needs to be ensured in the information cooperation between each of the business providers. Therefore, in the health service providing system 1 of the present embodiment, it is assumed that information cooperation to such an extent that an individual cannot be identified is performed between the business providers, and the gene information analysis provider 300 manages personal information of a user related to the health service (hereinafter, referred to as "user information"). As a unit for implementing such information management, the gene information analysis provider 300 includes a user information management apparatus 30 (information management device).

Note that, in Fig. 1, the gene information provider 200 is described as a business provider different from the gene information analysis provider 300, and this illustrates an example in which a sample of a user is not directly provided from the user but is provided via the medical institution 100. The gene information provider 200 and the gene information analysis provider 300 may be different business providers as illustrated in Fig. 1, or may be the same business provider.

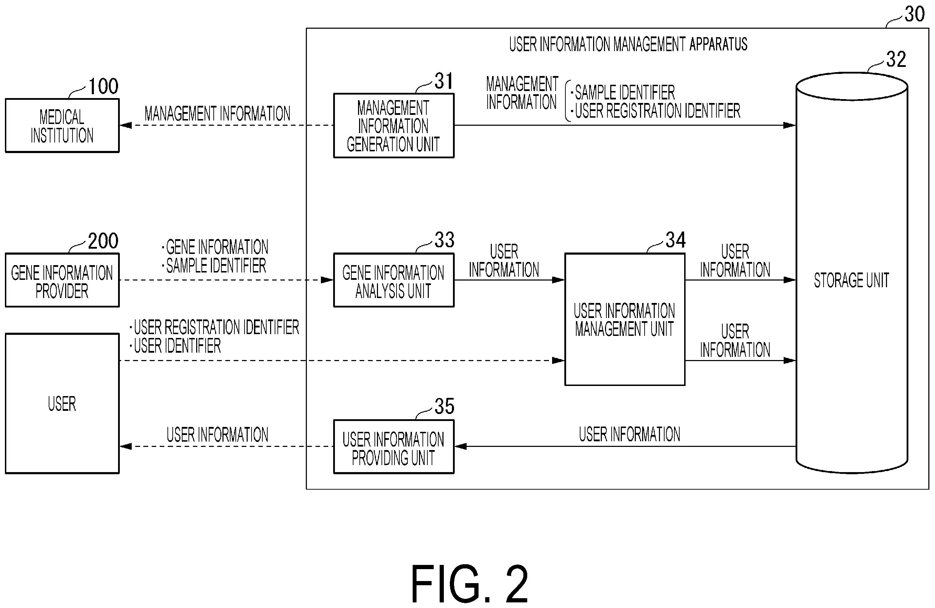

Fig. 2 is a block diagram illustrating a specific example of a functional configuration of the user information management apparatus 30 in the first embodiment. The user information management apparatus 30 includes a central processing unit (CPU), a memory, an auxiliary storage device, a communication interface, and the like connected by a bus, and executes a program. The user information management apparatus 30 functions as a device including a management information generation unit 31, a storage unit 32, a gene information analysis unit 33, a user information management unit 34, and a user information providing unit 35 by executing the program. Furthermore, the user information management apparatus 30 can exchange various types of information with the medical institution 100, the gene information provider 200, and a user by communication via the communication interface.

Note that, all or some of the functions of the user information management apparatus 30 may be implemented using hardware such as an application specific integrated circuit (ASIC), a programmable logic device (PLD), or a field programmable gate array (FPGA). The program may be recorded in a computer-readable recording medium. The computer-readable recording medium is a storage device such as, for example, a flexible disk, a magneto-optical disk, a read-only memory (ROM), a portable medium such as a compact disc read-only memory (CD-ROM), or a hard disk built in a computer system. The program may be transmitted via a telecommunication line.

The management information generation unit 31 generates management information for managing user information related to the health service. Specifically, the management information generation unit 31 issues a sample identifier for identifying a sample collected at a medical institution and a user registration identifier for identifying a user who has provided the sample. Here, the user registration identifier is identification information used when the user information management apparatus 30 associates a user with user information (hereinafter, referred to as "user registration"). The user registration identifier is generated as identification information different from the sample identifier. The management information generation unit 31 records the issued sample identifier and user information identifier in the storage unit 32 as a set of management information. On the other hand, the management information generation unit 31 provides the generated management information to the medical institution 100.

Note that, at the time of generation of management information, the management information is not associated with any sample or any user information. That is, at the time of generation of management information, the management information is merely a set of two different identifiers. The management information is associated with a specific user by user registration being performed in the user information management apparatus 30. Details of the user registration will be described below.

The storage unit 32 is formed using an auxiliary storage device such as a hard disk drive (HDD) or a solid state drive (SSD). The storage unit 32 stores sample management information and user information.

The gene information analysis unit 33 analyzes gene information provided from the gene information provider 200. The gene information analysis unit 33 outputs the gene information provided from the gene information provider 200 and information indicating the analysis result of the gene information to the user information management unit 34. For example, the gene information analysis unit 33 analyzes a physical constitution of a user on the basis of the gene information, and generates information, for example, related to change in behavior desired for the user for maintaining or improving health according to the physical constitution as user information.

For example, incidence rate of disease can be estimated as an example of a physical constitution of a user. For example, examples of a method of estimating incidence rate of disease on the basis of gene information include a method of constructing a risk model indicating relation between gene mutation and the incidence rate of disease (see, for example, Reference 1: "Quality of Scientific Evidence", https://mycode.jp/benefits/basis.html, MYCODE). In this case, a result of a case-control association analysis and results of the genome-wide association study can be used as sample data. Note that, in addition to the genetic factor, a statistical factor obtained by an epidemiological study or the like may be added to the risk model. For example, it is known that the incidence of esophageal cancer is greatly increased by a combination of a specific genetic factor and a specific lifestyle (for example, drinking or smoking) (see, for example, Reference 2: "Close Relation Between Alcohol, Tobacco, and Esophageal Cancer", https://biobankjp.org/cohort_1st/public/tsushin07/biobank_tsushin0 7_02.html, Biobank Report, 7th issue, August, 2009).

Furthermore, for example, in a case where an antibody test of COVID-19 is performed as the health service, it is conceivable to acquire biological information (for example, concentrations of various components in blood, and the like) from a sample instead of gene information, and analyze the biological information to estimate a physical constitution of a user. In this case, in the following embodiment, gene information can be replaced with biological information, and the analysis result of the biological information or information obtained on the basis of the analysis result can be replaced with user information.

The user information management unit 34 manages gene information and information indicating the analysis result output from the gene information analysis unit 33 as user information in association with a sample identifier provided together with the gene information.

Furthermore, on the other hand, the user information management unit 34 performs user registration in response to a request of a user. By this user registration, the user is associated with user information of the user. Specifically, the user registration is performed by identification information of the user used for user authentication (hereinafter, referred to as a "user identifier") being associated with a user registration identifier provided to the user. Note that the user registration identifier to be associated with a user identifier is provided from a user together with the user identifier at the time of a user registration request.

Here, since management information is generated as a set of a sample identifier and a user registration identifier, performing user registration corresponds to associating a user identifier with a sample identifier. Furthermore, since the sample identifier is associated with user information in advance, the user identifier can be associated with the user information by the user registration being performed.

Note that, the user identifier is identification information different from both a sample identifier and a user registration identifier. For example, the user identifier is information such as a user identification (ID) issued by a service use contract or the like. Furthermore, for example, the user identifier may be account information of a user used for login to a portal site described below. As described above, in a case where a user has identification information that has already been registered, the identification information may be used as a user identifier, and in a case where the user does not have identification information that has been registered, new identification information may be given as a user identifier. Furthermore, account information of a portal site different from the portal site described below may be used as a user identifier. In this case, the login to the portal site described below may be authenticated by an authentication function of the different portal site.

The user information providing unit 35 provides a user who has completed user registration (hereinafter, referred to as a "registered user") with user information acquired for the registered user.

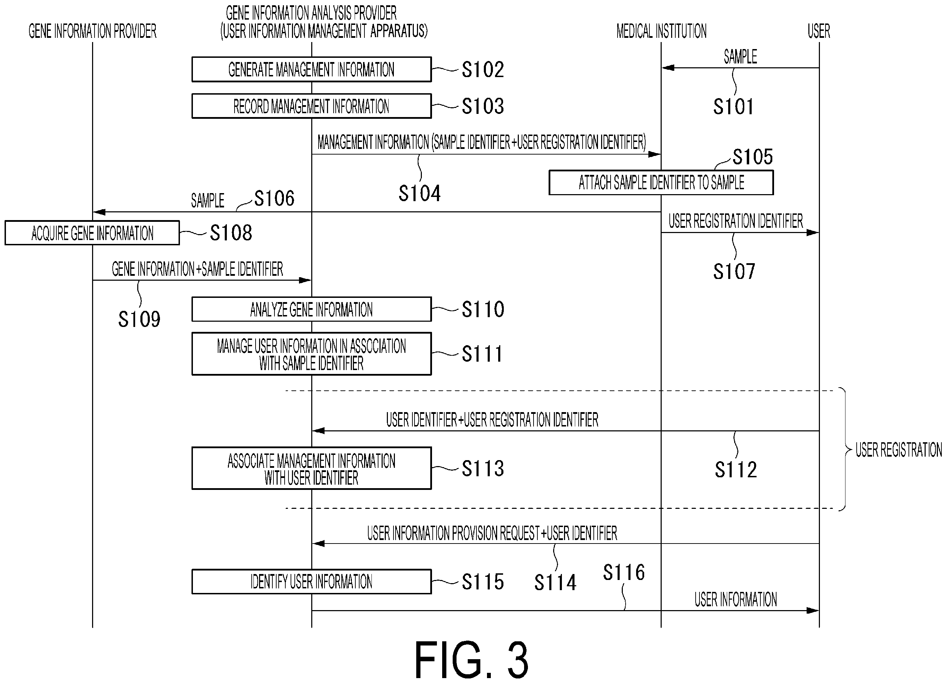

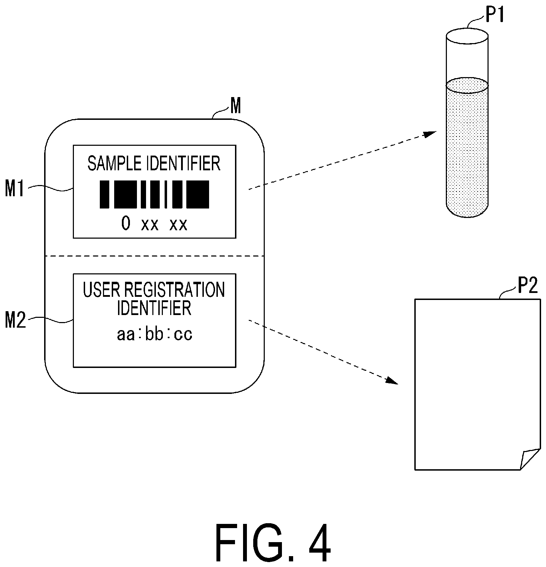

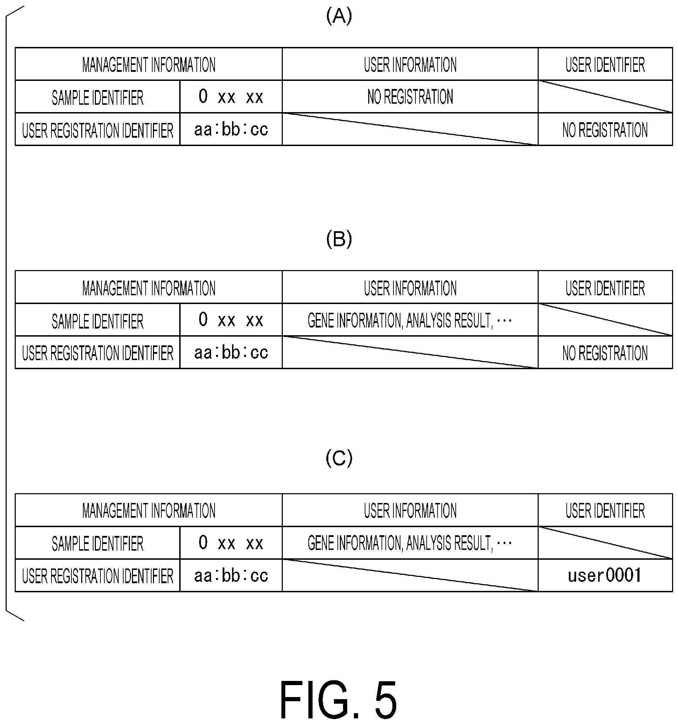

Fig. 3 is a sequence diagram illustrating a flow of processing and information cooperation from acquisition of a sample to provision of user information in the health service providing system 1 of the first embodiment. Furthermore, Figs. 4 and 5 are diagrams illustrating implementation examples of the processing and the information cooperation. Hereinafter, a flow of a series of the processing and the information cooperation illustrated in Fig. 3 will be described with appropriate reference to these implementation examples.

First, prior to or in parallel with provision of a sample from a user to the medical institution 100 (step S101), management information of the provided sample (that is, (a sample identifier and a user registration identifier) is generated (step S102). The management information generation unit 31 records the generated management information in the storage unit 32 (step S103) and provides the generated management information to the medical institution 100 (step S104).

For example, the management information is provided to the medical institution 100 in the form of a seal M1 on which the sample identifier is specified and a seal M2 on which the user registration identifier is specified (Fig. 4). Since the management information is represented by a set of the sample identifier and the user registration identifier, the seals M1 and M2 are provided, for example, in a state of being attached to one mount M so as not to be separated until a sample to be managed is generated.

Subsequently, when a sample to be managed is generated at the medical institution 100, the seal M1 indicating the sample identifier is attached to the sample P1 (step S105), and the sample P1 to which the seal M1 is attached is provided to the gene information provider 200 (step S106). For example, blood, saliva, or the like is collected as a sample. For example, blood can be used as a sample in a case where gene information is acquired or in a case where an antibody test is performed. Furthermore, for example, saliva can be used as a sample in a case where gene information is acquired. On the other hand, the seal M2 is provided to the user who has provided the sample (step S107). As described above, since the sample identifier and the user identifier are given to different targets, for example, perforations (broken line in the drawing) or the like may be provided on the mount M so that the seals M1 and M2 can be easily separated when the sample to be managed is generated. Furthermore, the seal M2 may be provided to the user with being attached to the mount M, or may be provided with being attached to a document P2 such as a medical examination application form or a consent form.

Subsequently, the gene information provider 200 acquires gene information using the sample provided from the medical institution 100 (step S108). For example, difference in genotype in which deoxyribonucleic acid (DNA) sequence is different by one base (commonly referred to as single nucleotide polymorphism (SNP)) is detected as gene information by a microarray or the like. The gene information of the user may be acquired for the entire genome, or may be acquired for the genome in a specific region. The gene information provider 200 provides the acquired gene information to the user information management apparatus 30 together with the sample identifier attached to the sample (step S109).

Subsequently, in the user information management apparatus 30, the gene information analysis unit 33 analyzes the gene information provided from the gene information provider 200 (step S110) to generate user information. The gene information analysis unit 33 manages the generated user information in association with the sample identifier provided together with the gene information (step Sill).

On the other hand, the user provided with the user registration identifier from the medical institution 100 performs user registration on the user information management apparatus 30. Specifically, the user presents the user registration identifier provided from the medical institution 100 and the user identifier that is identification information of the user to the user information management apparatus 30 (step S112). In the user information management apparatus 30, the user information management unit 34 identifies the management information of the user on the basis of the presented user registration identifier, and completes the user registration by associating the identified management information with the user identifier (step S113).

For example, the user information management unit 34 may be formed as a web server that provides each user with a portal site for providing user information. In this case, the user accesses his/her own portal site using an information communication terminal such as a smartphone or a personal computer (PC). At this time, for example, the user information management unit 34 may acquire a login ID input when the user logs in to the portal site as the user identifier, and provide a screen on which the user who has logged in inputs the user registration identifier used for the user registration.

The user information management unit 34 identifies the management information of the user on the basis of the user registration identifier acquired in this manner, and associates the user identifier with the identified management information. Fig. 5 is a diagram illustrating a flow of user registration. Fig. 5(A) illustrates a situation in which management information is not associated with any of user information and a user identifier. In this situation, the management information merely indicates a set of a sample identifier and a user registration identifier.

Thereafter, gene information is acquired on the basis of a collected sample, and the user information is generated on the basis of the acquired gene information. Fig. 5(B) illustrates a situation in which the generated user information is registered in the sample identifier provided together with the gene information.

Thereafter, a user provided with the user registration identifier from the medical institution 100 performs user registration on the user information management apparatus 30. Fig. 5(C) illustrates a situation in which the user registration identifier and the user identifier are further associated with each other from the situation of Fig. 5(B) by the user registration being performed. When the situation of Fig. 5(B) transitions to the situation of Fig. 5(C), the association between the user identifier and the user information is completed (that is, the user registration is completed). The user who has once completed the user registration can request the user information management apparatus 30 to provide the user information at any time thereafter by presenting the user identifier.

For example, in step S114, it is assumed that the user requests the user information management apparatus 30 to provide the user information. In this case, the user information providing unit 35 identifies user information to be provided on the basis of the presented user identifier and the management information of the user in response to the request (step S115), and provides the identified user information to the user who is the request source (step S116).

The user information management apparatus 30 of the first embodiment formed as described above includes the management information generation unit 31 (example of an identifier issuance unit) that issues a sample identifier for identifying a sample and a user registration identifier for identifying a user who has provided the sample, the storage unit 32 that stores a sample identifier and a user registration identifier issued by the management information generation unit 301 in association with each other, and a user information management unit 34 that associates user information related to a user who has provided a sample identified by the above sample identifier with the sample identifier in a case where the user information is provided together with the sample identifier, and associates a user identifier that is identification information of the user and is different from the above user registration identifier with user information by associating the user identifier with the user registration identifier in a case where the user identifier is provided together with the user registration identifier. With such a configuration, the user information management apparatus 30 of the first embodiment can provide more convenient health service.

Specifically, according to the user information management apparatus 30 of the first embodiment, since a user only needs to provide a sample to the medical institution 100, the user can more easily use the health service.

In addition, according to the user information management apparatus 30 of the first embodiment, information cooperation to such an extent that an individual cannot be identified is performed between the business providers, and the gene information analysis provider 300 manages user information, so that a user can use the health service with more security.

Note that, in the first embodiment, gene information provided from the gene information provider 200 and information indicating the analysis result are assumed as user information, but the user information may be any one of them. Furthermore, information other than gene information may be managed as user information as long as the information is provided together with a sample identifier. For example, in a case where, at the medical institution 100, a medical examination of a user who has provided a sample is performed and medical examination information indicating the result is provided to the user information management apparatus 30 together with a sample identifier of the sample, the user information management apparatus 30 may include the medical examination information in user information and manage the medical examination information. Furthermore, in this case, the user information management apparatus 30 may manage information obtained on the basis of the medical examination information as user information. In a case where an antibody test of COVID-19 is performed, the user information management apparatus 30 may manage the presence or absence of IgM antibodies or IgG antibodies detected from blood that is a sample by immunochromatography or the like as user information.

### <Second Embodiment>

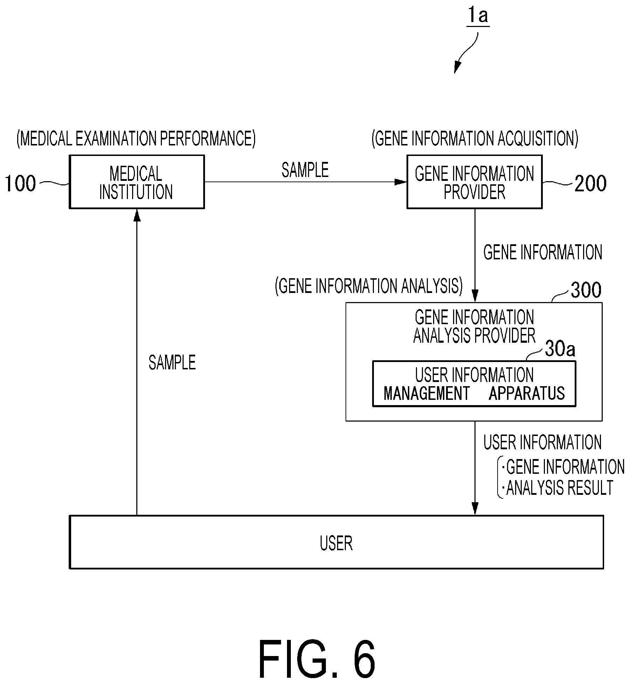

Fig. 6 is a diagram illustrating a specific example of a configuration of a health service providing system 1a in a second embodiment. The health service providing system 1a is different from the health service providing system 1 in the first embodiment in that a gene information analysis provider 300 includes a user information management apparatus 30a instead of the user information management apparatus 30. Other configurations of the health service providing system 1a are similar to those of the health service providing system 1 in the first embodiment. Therefore, the same reference signs as those in Fig. 1 are given to the same configurations, and detailed description thereof will be omitted.

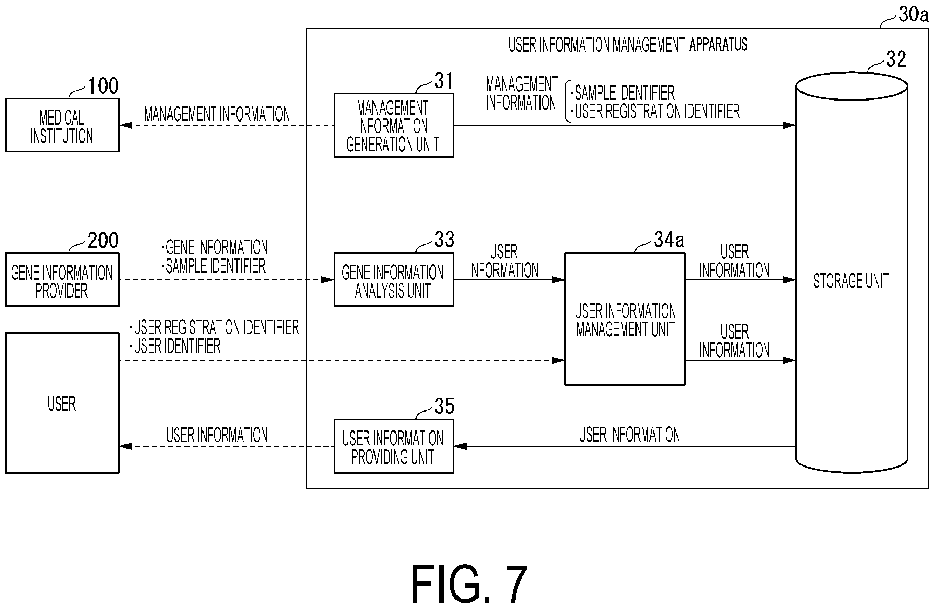

Fig. 7 is a block diagram illustrating a specific example of a functional configuration of the user information management apparatus 30a in the second embodiment. The user information management apparatus 30a is different from the user information management apparatus 30 in the first embodiment in including a user information management unit 34a instead of the user information management unit 34. Other configurations of the user information management apparatus 30a are similar to those of the user information management apparatus 30 in the first embodiment. Therefore, the same reference signs as those in Fig. 2 are given to the same configurations, and detailed description thereof will be omitted.

The user information management unit 34a is different from the user information management unit 34 in the first embodiment in having a function of invalidating a user registration identifier after completion of user registration in addition to the functions of the user information management unit 34 in the first embodiment. Specifically, the user information management unit 34a invalidates a user registration identifier of a user who has completed user registration by deleting the user registration identifier from management information.

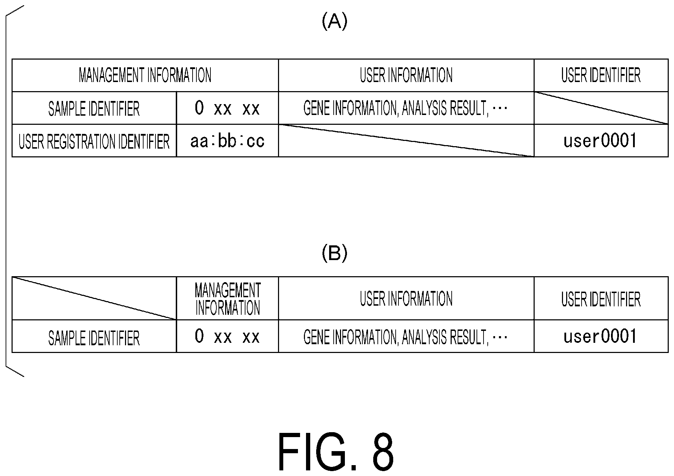

Fig. 8 is a diagram illustrating a flow of invalidation of a user registration identifier in the user information management apparatus 30a in the second embodiment. Fig. 8(A) illustrates a situation before the user registration identifier is invalidated, and illustrates a situation in which user registration has been once completed for a user identified by a user identifier "user001". In this case, for example, the user information management unit 34a associates the user identifier associated with the user registration identifier in Fig. 8(A) with a sample identifier, and then deletes the user registration identifier from management information (Fig. 8(B)).

In general, the greater the amount of identification information related to a user, the greater the possibility that personal information of the user is identified on the basis of any piece of identification information. In the health service providing system 1a of the present embodiment, a sample identifier and a user registration identifier are issued to associate a sample (and user information based on the sample) with a user who has provided the sample in the user information management apparatus 30a. Therefore, in a case where other user can physically acquire a medical examination application form, a consent form, or the like to which the user registration identifier is attached, the user information can be illicitly acquired by the user registration identifier being associated with a user identifier of the other user.

On the other hand, in the user information system 1a, the user information management unit 34a invalidates a user registration identifier of a user who has once completed user registration by deleting the user registration identifier from management information. As a result, a user registration identifier can be used as temporary information until user registration is completed, so that security of user information can be enhanced. Note that, in a case where personal information such as a date of birth or biometric information is separately acquired for a user who is a target for user registration, identity verification using these pieces of information may be performed at the time of user registration. As a result, in a case where an illicit user attempts user registration before the original user performs user registration, the act can be inhibited.

Note that, as another method of invalidating a user registration identifier, there is a method of setting a user registration identifier as information valid for a predetermined period. For example, it is conceivable that a management information generation unit 31 issues a user registration identifier as a one-time password with an expiration date. In this case, the user information management unit 34a may periodically check management information generated by the management information generation unit 31 and delete an expired user registration identifier from the management information including the user registration identifier. Furthermore, in this case, the user information management unit 34a may delete a user registration identifier, even within the expiration date, from management information at the timing when user registration is completed.

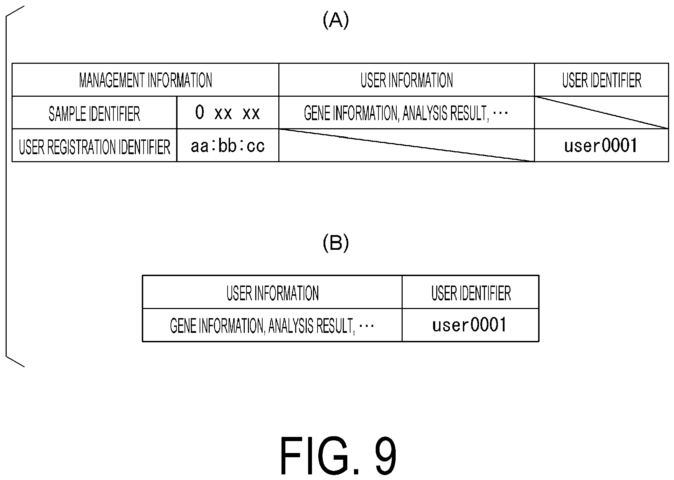

Furthermore, in a case where the gene information analysis provider 300 does not need to manage a sample identifier after completion of user registration, the user information management unit 34a may invalidate a sample identifier of a user who has once completed user registration by deleting the sample identifier from management information. The user information management unit 34a may invalidate either one of a sample identifier or a user registration identifier, or may invalidate both of them. For example, Fig. 9 is a diagram illustrating a flow of invalidation of both a sample identifier and a user registration identifier in the user information management apparatus of the second embodiment. In this case, the user information management unit 34a deletes management information after directly associating user information with a user identifier in Fig. 9(A) (Fig. 9(B)). Accordingly, the management information can be deleted from a storage unit 32 while correspondence between the user information and the user identifier is maintained.

Note that, in the first embodiment and the second embodiment, an example has been described in which the user information management apparatus is formed as one device, but the user information management apparatus may be implemented using a plurality of information processing devices communicably connected via a network. In this case, the functional units included in the user information management apparatus may be implemented in a distributed manner in the plurality of information processing devices.

As above, the embodiments of the present invention have been described in detail with reference to the drawings, however, the specific configuration is not limited to the embodiments, and includes design and the like without departing from the spirit of the present invention.

### Industrial Applicability

The present invention can be used as a service providing unit in which a plurality of business providers cooperatively provides personal information related to health of a user.

### Reference Signs List

- 1, 1a: Health service providing system
- 100: Medical institution
- 200: Gene information provider
- 300: Gene information analysis provider
- 30, 30a: User information management apparatus
- 31: Management information generation unit
- 32: Storage unit
- 33: Gene information analysis unit
- 34, 34a: User information management unit
- 35: User information providing unit

## Claims

1. An information management device comprising:
an identifier issuance unit that issues a sample identifier for identifying a sample and a user registration identifier for identifying a user who has provided the sample;
a storage unit that stores a sample identifier and a user registration identifier issued by the identifier issuance unit in association with each other; and
a user information management unit that associates user information related to a user who has provided a sample identified by the sample identifier with the sample identifier in a case where the user information is provided together with the sample identifier, and associates a user identifier that is identification information of the user and is different from the user registration identifier with user information by associating the user identifier with the user registration identifier in a case where the user identifier is provided together with the user registration identifier.

2. The information management device according to claim 1, wherein user information provided together with the sample identifier comprises gene information acquired on a basis of the sample.

3. The information management device according to claim 2 further comprising a gene information analysis unit that analyzes the gene information,
wherein the user information management unit associates information indicating an analysis result of the gene information with a user identifier.

4. The information management device according to any one of claims 1 to 3,
wherein user information provided together with the sample identifier comprises medical examination information indicating a result of a medical checkup of the user.

5. The information management device according to any one of claims 1 to 4,
wherein, in a case where a user identifier is associated with user information, the user information management unit deletes a user registration identifier of a user identified by the user identifier or a sample identifier corresponding to the user registration identifier from the identifier management unit.

6. The information management device according to any one of claims 1 to 5 further comprising an information providing unit that provides, in response to provision of a user identifier from a user who has associated a user identifier with user information, user information corresponding to the user identifier to the user.

7. An information management method comprising:
an identifier issuance step for issuing a sample identifier for identifying a sample and a user registration identifier for identifying a user who has provided the sample;
a storage step for storing a sample identifier and a user registration identifier issued in the identifier issuance step in association with each other; and
an information management step for associating user information related to a user who has provided a sample identified by the sample identifier with the sample identifier in a case where the user information is provided together with the sample identifier, and associating a user identifier that is identification information of the user and is different from the user registration identifier with user information by associating the user identifier with the user registration identifier in a case where the user identifier is provided together with the user registration identifier.

8. A computer program that causes a computer to execute steps, the steps comprising:
an identifier issuance step for issuing a sample identifier for identifying a sample and a user registration identifier for identifying a user who has provided the sample;
a storage step for storing a sample identifier and a user registration identifier issued in the identifier issuance step in association with each other; and
an information management step for associating user information related to a user who has provided a sample identified by the sample identifier with the sample identifier in a case where the user information is provided together with the sample identifier, and associating a user identifier that is identification information of the user and is different from the user registration identifier with user information by associating the user identifier with the user registration identifier in a case where the user identifier is provided together with the user registration identifier.
